# EUROPÄISCHE PATENTSCHRIFT

(11) **EP 0 723 574 B1**
(45) Veröffentlichungstag und Bekanntmachung des Hinweises auf die Patenterteilung: **02.06.1999**
(21) Anmeldenummer: 94928400.4
(22) Anmeldetag: 30.09.1994
(51) Int. Cl.: C10L 1/00, C10M 171/00, C10L 1/22, C09B 29/00, C09B 29/12, C09B 29/26, C09B 31/02, C09B 31/062, C09B 31/065, C09B 29/10, G01N 31/22

(54) **VERFAHREN ZUM NACHWEIS VON MARKIERTEN MINERALÖLEN**
PROCESS FOR DETECTING MARKED MINERAL OILS
PROCEDE DE DETECTION D'HUILES MINERALES MARQUEES

(30) Priorität: 12.10.1993 DE 4334678
(43) Veröffentlichungstag der Anmeldung: 31.07.1996
(73) Patentinhaber: BASF AKTIENGESELLSCHAFT, 67056 Ludwigshafen (DE)
(72) Erfinder: DYLLICK-BRENZINGER, Rainer, D-69469 Weinheim (DE); RAULFS, Friedrich-Wilhelm, D-67061 Ludwigshafen (DE); SCHLÖSSER, Ulrike, D-67071 Ludwigshafen (DE); BECK, Karin, Heidrun, D-67067 Ludwigshafen (DE); SCHOLZ, Gerhard, D-67059 Ludwigshafen (DE)
(86) Internationale Anmeldenummer: EP9403259
(87) Internationale Veröffentlichungsnummer: WO9510581

(56) Entgegenhaltungen:
- EP-A- 0 012 297
- EP-A- 0 031 572
- EP-A- 0 152 765
- EP-A- 0 509 818
- EP-A- 0 584 915
- FR-A- 2 141 667
- FR-A- 2 160 666
- GB-A- 905 321
- US-A- 3 056 642
- CHEMICAL ABSTRACTS, vol. 81, no. 10, 9. September 1974, Columbus, Ohio, US; abstract no. 51088q, 'EFFECT OF SUBSTITUENTS ON THE PROPERTIES OF DISPERSE AZO DYES WHICH ARE P-TERT-BUTYLPHENOL DERIVATIVES.' Seite 82-83 ;

## Beschreibung

Die vorliegende Erfindung betrifft ein neues Verfahren zum Nachweis von mit Azofarbstoffen markierten Mineralölen sowie die Verwendung von Monoazofarbstoffen als Markierungsmittel für Mineralöle.

Aus der US-A-5 156 653 ist die Verwendung von Phenylazophenolen zum Markieren von Mineralölen bekannt. Der Nachweis der Markierungsmittel wird dort durch Schütteln des markierten Mineralöls mit einer Mischung aus Wasser, Diethylenglykol und Methoxyethoxypropylamin durchgeführt, wobei sich eine Mineralölphase und eine wäßrige Phase, die den Azofarbstoff enthält, ausbildet. Es hat sich jedoch gezeigt, daß die dort beschriebene Nachweismethode nicht zufriedenstellend ist. Sie kann nicht allgemein angewendet werden und ergibt öfters nur eine äußerst schwache Anfärbung der wäßrigen Phase, weil ein Teil des verwendeten Farbstoffs in der Ölphase bleibt.

Die US-A-3 764 273 beschreibt die Extraktion von Chinizarinderivaten sowie von speziellen Azofarbstoffen u.a. mit wäßriger Lauge und Dimethylsulfoxid.

Aus der GB-A-905 321, Chemical Abstracts, Band 81, 1974, Nr. 51088q sowie FR-A-2 160 666 sind Azofarbstoffe der im folgenden zitierten Formel Ia bekannt. Die EP-A-12 297, EP-A-31 572, US-A-3 056 642 sowie die obengenannte FR-A-2 160 666 beschreiben die Verwendung von Azofarbstoffen zum Einfärben von Mineralölen.

Aufgabe der vorliegenden Erfindung war es nun, ein neues Verfahren zum Nachweis von durch Azofarbstoffe markierten Mineralölen bereitzustellen, das einfach durchzuführen ist und mittels dessen die Markierungsmittel auch noch in geringer Konzentration durch eine starke Farbreaktion sichtbar gemacht werden können.

Es wurde nun gefunden, daß der Nachweis von mit Azofarbstoffen markierten Mineralölen, indem man das markierte Mineralöl mit einem Extraktionsmittel, enthaltend Wasser, ein Lösungsmittel und Base, behandelt, wobei der Azofarbstoff vom Mineralöl in die wäßrige Phase übertritt, vorteilhaft gelingt, wenn man als Markierungsmittel einen Azofarbstoff der Formel Ia 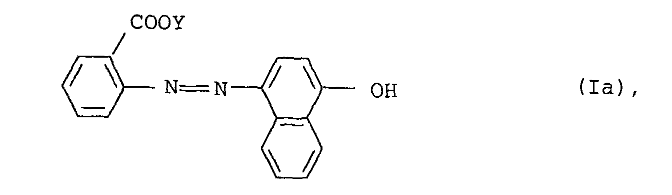 in der
- Y: C₇-C₁₁-Alkyl bedeutet,
oder der Formel Ib 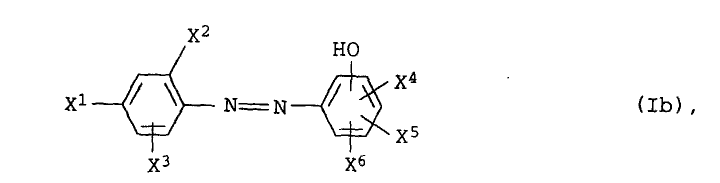 in der
- X¹: Wasserstoff oder durch C₁-C₄-Alkyl substituiertes Phenylazo,
- X²: Wasserstoff,
- X³: Wasserstoff oder C₁-C₄-Alkyl,
- X⁴: Wasserstoff, C₁-C₄-Alkyl, C₁-C₄-Dialkylamino oder C₁-C₁₆-Monoalkylamino,
- X⁵: Wasserstoff oder C₁-C₄-Alkyl und
- X⁶: Wasserstoff, C₁-C₄-Alkyl oder C₁-C₄-Alkoxy bedeuten,
mit der Maßgabe, daß a) mindestens einer der drei Reste X¹, X² und X³ von Wasserstoff verschieden ist, b) X² auch Cyano bedeutet, wenn X⁴ für C₁-C₄-Dialkylamino steht, c) X² auch C₁-C₄-Alkoxycarbonyl bedeutet, wenn alle Reste X⁴, X⁵ und X⁶ von Wasserstoff verschieden sind und d) einer der Reste X¹, X² oder X³ auch Nitro bedeutet, wenn X⁴ für C₁-C₁₆-Monoalkylamino und X⁶ für C₁-C₄-Alkyl stehen,
als Lösungsmittel ein mit Wasser teilweise oder vollständig mischbares organisches Lösungsmittel und als Base ein Alkali- oder Erdalkalihydroxid, ein Alkalicarbonat oder eine Ammoniumverbindung der Formel II 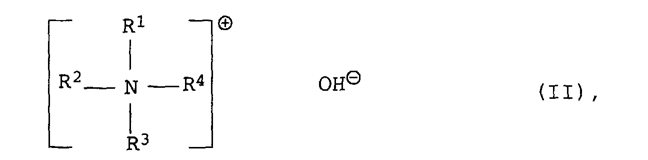 worin R¹, R², R³ und R⁴ unabhängig voneinander jeweils C₁-C₁₆-Alkyl oder Benzyl bedeuten, verwendet.

Alle in der obengenannten Formel I auftretenden Alkylreste können sowohl geradkettig als auch verzweigt sein.

Sofern nicht anders vermerkt, können, wenn in der obengenannten Formel I substituierte Phenyl- oder Naphthylgruppen auftreten, diese auch gleichzeitig verschiedene Substituenten an einem Phenyl- oder Naphthylring aufweisen.

Reste X², X³, X⁴, X⁵, und X⁶ sind z.B. Methyl, Ethyl, Propyl, Isopropyl, Butyl, Isobutyl oder sec-Butyl.

Reste X⁴ sind weiterhin z.B. Pentyl, Isopentyl, Neopentyl, tert-Pentyl, Hexyl, 2-Methylpentyl, Heptyl, Octyl, 2-Ethylhexyl oder Isooctyl.

Reste R¹, R², R³ und R⁴ sind weiterhin z.B. Heptyl, Octyl, 2-Ethylhexyl, Isooctyl, Nonyl, Isononyl, Decyl, Isodecyl oder Undecyl (die obigen Bezeichnungen Isooctyl, Isononyl und Isodecyl sind Trivialbezeichnungen und stammen von den nach der Oxosynthese erhaltenen Alkoholen - vgl. dazu Ullmanns Encyclopädie der technischen Chemie, 4. Auflage, Band 7, Seiten 215 bis 217, sowie Band 11, Seiten 435 und 436).

Reste X⁴ sind weiterhin z.B. Dimethylamino, Diethylamino, Dipropylamino, Diisopropylamino, Dibutylamino, Methylamino, Ethylamino, Propylamino, Isopropylamino, Butylamino, Isobutylamino, sec-Butylamino, Pentylamino, Isopentylamino, Neopentylamino, tert-Pentylamino, Hexylamino, 2-Methylpentylamino, Heptylamino, Octylamino, 2-Ethylhexylamino, Isooctylamino, Nonylamino, Isononylamino, Decylamino, Isodecylamino, Undecylamino, Dodecylamino, Tridecylamino, 3,5,5,7-Tetramethylnonylamino, Isotridecylamino, Tetradecylamino, Pentadecylamino, Hexadecylamino, Benzyl oder 1- oder 2-Phenylethyl.

Reste X¹ sind weiterhin z.B. Phenylazo, 2-, 3- oder 4-Methylphenylazo oder 2,4- oder 2,6-Dimethylphenylazo.

Reste X², X⁴ und X⁶ sind weiterhin z.B. Methoxy, Ethoxy, Propoxy, Isopropoxy, Butoxy, Isobutoxy oder sec-Butoxy.

Reste X² sind weiterhin z.B. Methoxycarbonyl, Ethoxycarbonyl, Propoxycarbonyl, Isopropoxycarbonyl, Butoxycarbonyl, Isobutoxycarbonyl, sec-Butoxycarbonyl, Pentyloxycarbonyl, Isopentyloxycarbonyl, Neopentyloxycarbonyl, tert-Pentyloxycarbonyl, Hexyloxycarbonyl, 2-Methylpentyloxycarbonyl, Heptyloxycarbonyl, Octyloxycarbonyl, 2-Ethylhexyloxycarbonyl, Isooctyloxycarbonyl, Nonyloxycarbonyl, Isononyloxycarbonyl, Decyloxycarbonyl, Isodecyloxycarbonyl, Undecyloxycarbonyl, Dodecyloxycarbonyl, Tridecyloxycarbonyl, Isotridecyloxycarbonyl, Tetradecyloxycarbonyl, Pentadecyloxycarbonyl oder Hexadecyloxycarbonyl.

Geeignete Alkali- oder Erdalkalihydroxide oder Alkalicarbonate, die im erfindungsgemäßen Verfahren zur Anwendung gelangen können, sind z.B. Lithium-, Natrium-, Kalium-, Magnesium- oder Calciumhydroxid oder Lithium-, Natrium- oder Kaliumcarbonat.

Geeignete Ammoniumverbindungen der Formel II sind z.B. Tetramethylammoniumhydroxid, Tetraethylammoniumhydroxid, Tetrapropylammoniumhydroxid, Tetrabutylammoniumhydroxid, Benzyltrimethylammoniumhydroxid, Benzyltriethylammoniumhydroxid oder Benzyltributylammoniumhydroxid.

Die Anwendung von Alkali- oder Erdalkalihydroxiden als Base ist bevorzugt, wobei Alkalihydroxide besonders hervorzuheben sind.

Besonders bevorzugt ist die Anwendung von Lithium-, Natrium- oder Kaliumhydroxid als Base, wobei Natriumhydroxid besondere Bedeutung zukommt.

Da das Extraktionsmittel auch Wasser enthält, werden die Alkali- oder Erdalkalihydroxide, Alkalicarbonate oder Ammoniumverbindungen der Formel II, vorzugsweise direkt als wäßrige Lösung angewandt, wobei diese in der Regel einen Gehalt an Alkali- oder Erdalkalihydroxid, Alkalicarbonat oder Ammoniumverbindungen der Formel II von 0,1 bis 10 Gew.-%, vorzugsweise 1 bis 10 Gew.-%, jeweils bezogen auf das Gewicht der wäßrigen Lösung, aufweisen.

Geeignete Lösungsmittel sind mit Wasser teilweise oder vollständig mischbare organische Lösungsmittel. Dies sind z.B. Säureamide, insbesondere Carbonsäureamide, wie Formamid, N-Methylformamid, N,N-Dimethylformamid, N,N-Diethylformamid, N,N-Dimethylacetamid, Pyrrolidin-2-on, 1-Methylpyrrolidin-2-on (NMP), 1,3-Dimethylhexahydropyrimid-2-on oder Hexamethylphosphonsäuretriamid, N,N,N',N'-Tetramethylguanidin, Acetonitril, Nitromethan, Amine, wie 2-Aminoethanol, 1,10-Diamino-4,7-dioxadecan, 1,13-Diamino-4,7,10-trioxatridecan, 2- oder 3-Methoxypropylamin, 1,8-Bis(dimethylamino)naphthalin oder ein Amin der Formel H₂N-CH(CH₃)CH₂[-OCH(CH₃)CH₂]_{2,6}-OCH₂CH(CH₃)-NH₂, 1-Methylpyrazol, Dimethylsulfoxid (DMSO) oder Sulfolan.

Bevorzugte Lösungsmittel sind 1-Methylpyrazol, Pyrrolidin-2-on, 1-Methylpyrrolidin-2-on, Dimethylsulfoxid oder Sulfolan.

In manchen Fällen kann es auch von Vorteil sein, wenn das Extraktionsmittel weitere Zusatzstoffe, z.B. 4-Nonylphenol, enthält oder wenn man dem Mineralöl vor der Extraktion 4-Nonylphenol zusetzt.

Bezogen auf das Gewicht des Extraktionsmittels beträgt der Anteil an Lösungsmittel in der Regel 50 bis 99 Gew.-%, vorzugsweise 90 bis 99 Gew.-%.

Zum Markieren von Mineralöl werden die Azofarbstoffe der Formel Ia oder Ib entweder in Substanz oder in Form von Lösungen angewandt. Als Lösungsmittel eignen sich vorzugsweise aromatische Kohlenwasserstoffe, wie Toluol, Xylol, Dodecylbenzol, Diisopropylnaphthalin oder ein Gemisch höherer Aromaten, das unter dem Namen Shellsol® AB (Fa. Shell) handelsüblich ist. Üblicherweise können zur Verbesserung der Löslichkeit noch weitere Cosolventien, z.B. Alkohole, wie Methanol, Ethanol, Propanol, Isopropanol, Butanol, Isobutanol, Pentanol, Hexanol, Heptanol, Octanol, 2-Ethylhexanol oder Cyclohexanol, Glykole, wie Butylethylenglykol oder Methylpropylenglykol, Amine, wie Triethylamin, Diisooctylamin, Dicyclohexylamin, Anilin, N-Methylanilin, N,N-Dimethylanilin, Toluidin oder Xylidin, Alkanolamine, wie 3-(2-Methoxyethoxy)propylamin, o-Kresol, m-Kresol, p-Kresol, Ketone, wie Diethylketon oder Cyclohexanon, Lactame, wie γ-Butyrolactam, Carbonate, wie Ethylencarbonat oder Propylencarbonat, Phenole, wie t-Butylphenol oder Nonylphenol, Ester, wie Phthalsäuremethylester, Phthalsäureethylester, Phthalsäure-(2-ethylhexyl)ester, Essigsäureethylester, Essigsäurebutylester oder Essigsäurecyclohexylester, Amide, wie N,N-Dimethylformamid, N,N-Diethylacetamid oder N-Methylpyrrolidin-2-on, oder deren Mischungen verwendet werden. Um eine hohe Viskosität der resultierenden Lösungen zu vermeiden, wählt man im allgemeinen eine Konzentration an Azofarbstoff Ia oder Ib von 1 bis 50 Gew.-%, vorzugsweise 10 bis 50 Gew.-%, jeweils bezogen auf die Lösung.

Bei der Behandlung des markierten Mineralöls mit dem Extraktionsmittel, beispielsweise durch Schütteln, tritt der Azofarbstoff der Formel Ia oder Ib in die wäßrige Phase über, die dabei deutlich sichtbar gefärbt wird.

Mittels des erfindungsgemäßen Verfahrens gelingt es sehr einfach, die mit den Azofarbstoffen der Formel Ia oder Ib markierten Mineralöle nachzuweisen. Ein weiterer Vorteil des erfindungsgemäßen Verfahrens besteht darin, daß auch Farbstoffe extrahierbar sind, die nur wenig acide sind.

Ein weiterer Gegenstand der vorliegenden Erfindung ist die Verwendung von Azofarbstoffen der obengenannten Formel Ia zum Markieren von Mineralölen.

Unter Mineralölen im erfindungsgemäßen Sinn sind beispielsweise Treibstoffe, wie Benzin, Kerosin oder Dieselöl, oder Öle, wie Heizöl oder Motorenöl, zu verstehen.

Die Azofarbstoffe der Formel Ia oder Ib eignen sich insbesondere zum Markieren von Mineralölen, bei denen eine Kennzeichnung gefordert wird, z.B. aus steuerlichen Gründen. Um die Kosten der Kennzeichnung gering zu halten, strebt man dabei an, für die Markierung möglichst geringe Mengen an Markierungsmittel anzuwenden.

Unter Markierung im erfindungsgemäßen Sinn ist ein Zusatz der Azofarbstoffe der Formel Ia oder Ib in solcher Konzentration zu Mineralölen zu verstehen, daß die Mineralöle dadurch für das menschliche Auge entweder überhaupt nicht oder nur wenig sichtbar angefärbt sind, wobei jedoch die Farbstoffe der Formel Ia und Ib durch die hier näher beschriebenen Nachweismethoden leicht und deutlich sichtbar detektierbar sind.

Zum Markieren von Mineralöl werden die Azofarbstoffe der Formel Ia oder Ib, wie oben bereits ausgeführt, entweder in Substanz oder in Form von Lösungen angewandt.

Die erfindungsgemäß anzuwendenden Azofarbstoffe der Formel Ia oder Ib haben den Vorteil, daß sie, abhängig von ihrer Konzentration, geeignet sind, Mineralöle gut sichtbar einzufärben und/ oder als Markierungssubstanzen angewandt werden können.

Mittels den erfindungsgemäß anzuwendenden Azofarbstoffen der Formel Ia oder Ib gelingt es sehr einfach, markierte Mineralöle nachzuweisen, selbst wenn die Markierungssubstanzen nur in einer Konzentration von ungefähr 10 ppm oder darunter vorliegen.

Die Azofarbstoffe der Formel Ia haben bei ihrer Anwendung den Vorteil, daß sie über eine sehr gute Löslichkeit verfügen und auch gut verdünnbar sind. Außerdem können mit ihnen bei der Nachweisreaktion deutlich bathochromere Farbtöne erzielt werden als mit den aus der US-A-5 156 653 bekannten Farbstoffen.

Die Azofarbstoffe der Formel Ib können nach an sich bekannten Methoden erhalten werden. Beispielsweise kann man ein Anilin der Formel III 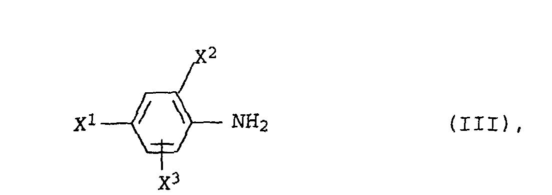 in der X¹, X² und X³ jeweils die obengenannte Bedeutung besitzen, auf üblichem Wege diazotieren und die resultierende Diazoniumverbindung mit einem Phenol der Formel IV 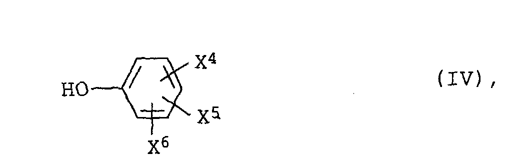 in der X⁴, X⁵ und X⁶ jeweils die obengenannte Bedeutung besitzen, kuppeln.

Bei den weiteren Azofarbstoffen der Formel Ia handelt es sich in der Regel um an sich bekannte Verbindungen. Sie sind z.B. in der US-A-5 156 653 oder der US-A-4 473 376 beschrieben oder können nach den dort genannten Methoden erhalten werden.

Die folgenden Beispiele sollen die Erfindung näher erläutern. (Die dort genannten Teile sind jeweils Gewichtsteile).

### Beispiel 1

Handelsübliches Dieselöl wurde mit 10 ppm eines Farbstoffs (F1) der Formel 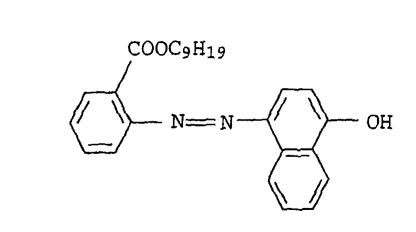 versetzt.

9 Teile des so rot markierten Dieselöls wurden mit 0,5 Teilen einer Mischung aus 4 Teilen dest. Wasser, 4 Teilen 1,10-Diamino-4,7-dioxadecan und 2 Teilen 10 gew.-%iger Natronlauge versetzt und kräftig geschüttelt. Nach ca. 1 min setzte sich eine wäßrige Phase, die stark rot gefärbt war, unten ab. Die überstehende Dieselölphase war fast farblos. Die Färbung der wäßrigen Phase war auch nach 24 h bei Raumtemperatur unverändert.

Ein unter analogen Bedingungen durchgeführter Blindversuch mit unmarkiertem Dieselöl ergab eine wäßrige Phase, die fast farblos war.

Die durch Durchführung der Reaktion mit einem mit dem Farbstoff F1 markierten handelsüblichen Benzin (Fa. Aral) unter analogen Bedingungen, ergab den gleichen Farbeffekt wie bei markiertem Dieselöl.

Ein unter analogen Bedingungen durchgeführter Blindversuch mit unmarkiertem Benzin (Fa. Aral) ergab eine wäßrige Phase, die fast farblos war.

In analoger Weise wurde Dieselöl, das mit den im folgenden aufgeführten Farbstoffen markiert war, mit verschiedenen Reagenzlösungen, die im folgenden ebenfalls aufgeführt sind, nachgewiesen.

### Farbstoffe:

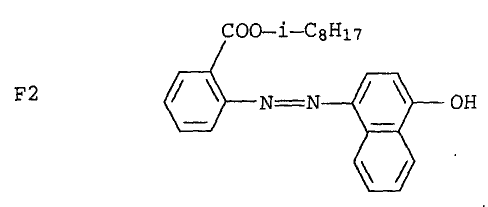

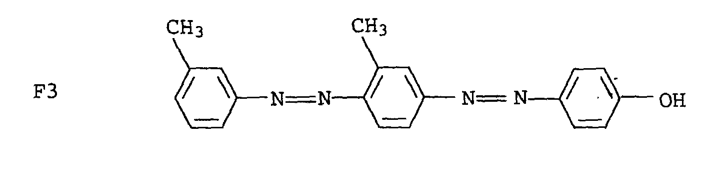

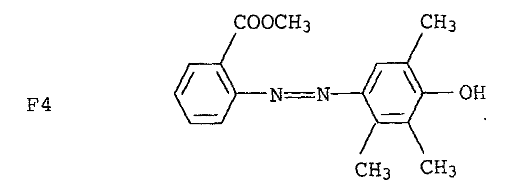

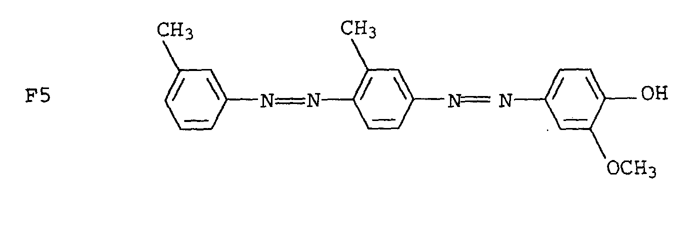

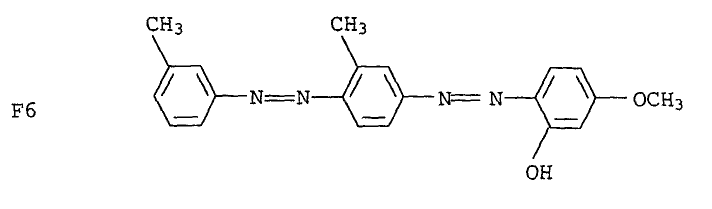

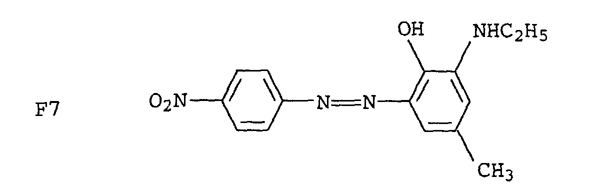

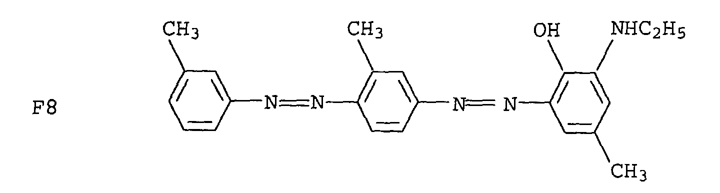

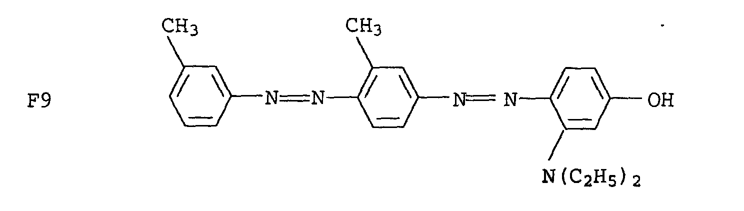

### Reagenz lösungen:

- R1: 9 Teile NMP und 1 Teil 1 gew.-%ige wäßrige Lithiumhydroxidlösung
- R2: 9 Teile Pyrrolidin-2-on und 1 Teil 1 gew.-%ige wäßrige Lithiumhydroxidlösung
- R3: 9 Teile DMSO und 1 Teil 2 gew.-%ige wäßrige Lithiumhydroxidlösung
- R4: 9,5 Teile 1-Methylimidazol und 0,5 Teile 5 gew.-%ige wäßrige Lithiumhydroxidlösung
- R5: 9,5 Teile Amin der Formel H₂N-CH(CH₃)CH₂[-OCH(CH₃)CH₂]_{2,6}-OCH₂CH(CH₃)-NH₂ und 0,5 Teile 1 gew.-%ige wäßrige Lithiumhydroxidlösung
- R6: 9 Teile 1,3-Dimethylhexahydropyrimid-2-on und 1 Teil 1 gew.-%ige wäßrige Lithiumhydroxidlösung
- R7: 9 Teile 1,13-Diamino-4,7,10-trioxatridecan und 1 Teil 1 gew.-%ige wäßrige Lithiumhydroxidlösung
- R8: 5 Teile 3-Methoxypropylamin und 1 Teil 5 gew.-%ige wäßrige Kalilauge
- R9: 4 Teile dest. Wasser, 4 Teile 1,10-Diamino-4,7-dioxadecan und 2 Teile 10 gew.-%ige Natronlauge
- R10: 9 Teile DMSO, 1 Teil 2 gew.-%ige wäßrige Lithiumhydroxidlösung und 1 Teil 4-Nonylphenol

Die folgenden Ergebnisse wurden erzielt.

| Bsp. Nr. | Farbstoff | Reagenz | Farbton der wäßrigen Phase | Farbton der öligen Phase nach dem Schütteln | Farbton der öligen Phase vor dem Schütteln | Markerkonzentration in Dieselöl |
|---|---|---|---|---|---|---|
| 2 | F1 | R1 | stark rot | farblos | stark gelb | 10 ppm |
| 3 | F1 | R2 | stark rot | farblos | stark gelb | 10 ppm |
| 4 | F1 | R3 | stark rot | farblos | stark gelb | 10 ppm |
| 5 | F1 | R4 | stark rot | farblos | stark gelb | 10 ppm |
| 6 | F1 | R8 | schwach rot | schwach gelb | stark gelb | 10 ppm |
| 7 | F1 | R10 | stark rot | fast farblos | stark gelb | 10 ppm |
| 8 | F2 | R1 | stark orange | farblos | stark gelb | 10 ppm |
| 9 | F2 | R2 | stark orange | farblos | stark gelb | 10 ppm |
| 10 | F2 | R3 | stark orange | fast farblos | stark gelb | 10 ppm |
| 11 | F2 | R8 | stark rot | fast farblos | stark gelb | 10 ppm |
| 12 | F2 | R9 | stark rot | schwach gelb | stark gelb | 10 ppm |
| 13 | F2 | R10 | stark rot | fast farblos | stark gelb | 10 ppm |
| 14 | F3 | R1 | stark rot | leicht gelb | stark gelb | 5 ppm |
| 15 | F3 | R2 | stark rot | fast farblos | stark gelb | 5 ppm |
| 16 | F3 | R3 | stark rot-violett | fast farblos | stark gelb | 5 ppm |
| 17 | F3 | R8 | stark rot | fast farblos | stark gelb | 5 ppm |
| 18 | F3 | R9 | stark rot | schwach gelb | stark gelb | 5 ppm |
| 19 | F3 | R10 | stark rot | fast farblos | stark gelb | 5 ppm |
| 20 | F4 | R1 | orange | schwach gelb | gelb | 5 ppm |
| 21 | F4 | R2 | stark orange | schwach orange | stark gelb | 5 ppm |
| 22 | F4 | R3 | stark orange | fast farblos | stark gelb | 5 ppm |
| 23 | F4 | R4 | stark rot | fast farblos | stark gelb | 5 ppm |
| 24 | F4 | R5 | stark orange | schwach orange | stark gelb | 5 ppm |
| 25 | F4 | R6 | stark orange | schwach orange | stark gelb | 5 ppm |
| 26 | F4 | R7 | stark orange | schwach orange | stark gelb | 5 ppm |
| 27 | F4 | R8 | orange | schwach orange | stark gelb | 5 ppm |
| 28 | F4 | R9 | stark hellrot | schwach gelb | stark gelb | 10 ppm |
| 29 | F4 | R10 | stark rot | schwach gelb | stark gelb | 10 ppm |
| 30 | F5 | R1 | stark violett | fast farblos | stark gelb | 5 ppm |
| 31 | F5 | R2 | stark violett | fast farblos | stark gelb | 5 ppm |
| 32 | F5 | R3 | stark dunkelblau | fast farblos | stark gelb | 5 ppm |
| 33 | F5 | R4 | stark grün | schwach grün | stark gelb | 5 ppm |
| 34 | F5 | R5 | stark violett | schwach rot | stark gelb | 5 ppm |
| 35 | F5 | R6 | stark violett | fast farblos | stark gelb | 5 ppm |
| 36 | F5 | R7 | stark blau-grau | fast farblos | stark gelb | 5 ppm |
| 37 | F5 | R8 | stark rot-violett | fast farblos | stark gelb | 5 ppm |
| 38 | F5 | R9 | stark rot | schwach gelb | stark gelb | 5 ppm |
| 39 | F5 | R10 | stark violett | fast farblos | stark gelb | 5 ppm |
| 40 | F6 | R1 | stark violett | fast farblos | stark gelb | 5 ppm |
| 41 | F6 | R2 | stark gelb-orange | schwach gelb | stark gelb | 5 ppm |
| 42 | F6 | R3 | stark violett | fast farblos | stark gelb | .5 ppm |
| 43 | F6 | R4 | stark orange | schwach gelb | stark gelb | 5 ppm |
| 44 | F6 | R5 | stark orange | schwach gelb | stark gelb | 5 ppm |
| 45 | F6 | R6 | stark orange | schwach gelb | stark gelb | 5 ppm |
| 46 | F6 | R7 | stark orange | schwach gelb | stark gelb | 5 ppm |
| 47 | F6 | R8 | stark orange | schwach gelb | stark gelb | 5 ppm |
| 48 | F6 | R9 | stark rot | schwach gelb | stark gelb | 5 ppm |
| 49 | F6 | R10 | stark dunkelrot | schwach gelb | stark gelb | 5 ppm |
| 50 | F7 | R1 | stark rot-violett | fast farblos | stark gelb | 5 ppm |
| 51 | F7 | R2 | stark violett | fast farblos | stark gelb | 5 ppm |
| 52 | F7 | R3 | stark violett | fast farblos | stark gelb | 5 ppm |
| 53 | F7 | R4 | stark grün | fast farblos | stark gelb | 5 ppm |
| 54 | F7 | R5 | stark rot-violett | fast farblos | stark gelb | 5 ppm |
| 55 | F7 | R6 | stark dunkelblau | fast farblos | stark gelb | 5 ppm |
| 56 | F7 | R7 | stark rot-violett | fast farblos | stark gelb | 5 ppm |
| 57 | F7 | R8 | stark hellblau | fast farblos | stark gelb | 5 ppm |
| 58 | F7 | R9 | stark hellblau | schwach gelb | stark gelb | 5 ppm |
| 59 | F7 | R10 | stark violett | schwach gelb | stark gelb | 5 ppm |
| 60 | F8 | R1 | stark gelb-braun | schwach gelb | stark orange | 5 ppm |
| 61 | F8 | R2 | stark rot-braun | schwach gelb | stark orange | 5 ppm |
| 62 | F8 | R3 | stark rot-violett | schwach gelb | stark orange | 5 ppm |
| 63 | F8 | R4 | stark gelb | schwach gelb | stark orange | 5 ppm |
| 64 | F8 | R5 | stark rot | schwach gelb | stark orange | 5 ppm |
| 65 | F8 | R6 | stark gelb-grün | schwach gelb | stark orange | 5 ppm |
| 66 | F8 | R7 | gelb-braun | schwach gelb | stark orange | 5 ppm |
| 67 | F8 | R8 | grün-grau | schwach gelb | stark orange | 5 ppm |
| 68 | F8 | R9 | farblos | schwach gelb-orange | stark orange | 5 ppm |
| 69 | F8 | R10 | stark rot-braun | schwach gelb | stark orange | 5 ppm |
| 70 | F9 | R1 | stark orange | schwach gelb | stark gelb | 5 ppm |
| 71 | F9 | R2 | stark orange | schwach gelb | stark gelb | 5 ppm |
| 72 | F9 | R3 | stark orange | schwach gelb | stark gelb | 5 ppm |
| 73 | F9 | R4 | stark gelb | schwach gelb | stark gelb | 5 ppm |
| 74 | F9 | R5 | stark orange | schwach gelb | stark gelb | 5 ppm |
| 75 | F9 | R6 | stark orange | schwach gelb | stark gelb | 5 ppm |
| 76 | F9 | R7 | stark orange | schwach gelb | stark gelb | 5 ppm |
| 77 | F9 | R8 | stark orange | schwach gelb | stark gelb | 5 ppm |
| 78 | F9 | R9 | stark orange | schwach gelb | stark gelb | 5 ppm |

### Beispiel 79 (Herstellung von F3)

a) 42,6 g (0,04 mol) m-Aminoazotoluol-hydrochlorid (24,5 gew.-%ig) und 0,2 g eines Netzmittels wurden in 32 ml Wasser und 12 ml 30 gew.-%iger Salzsäure suspendiert. Anschließend wurde mit 20 g Eis, 7 ml Toluol und 12 ml 23 gew.-%iger wäßriger Natriumnitritlösung versetzt und 2 h bei 5 bis 10°C gerührt.
b) 3,76 g (0,04 mol) Phenol und 100 ml Wasser wurden durch Zugabe von 50 gew.-%iger Natronlauge auf einen pH-Wert von 6,4 gestellt und bei 0°C mit der unter a) beschriebenen Diazoniumsalzlösung versetzt. Der pH-Wert wurde mit 10 gew.-%iger Natronlauge zwischen 4 und 5 gehalten. Nach Rühren über Nacht wurde mit 10 gew.-%iger Salzsäure der pH-Wert auf 1,5 erniedrigt und der ausgefallene Feststoff isoliert.
Ausbeute: 10,1 g

### Beispiel 80 (Herstellung von F4)

a) 3,0 g (0,02 mol) Anthranilsäuremethylester wurden in 30 ml Wasser, 50 g Eis und 5 ml konzentrierter Salzsäure bei 0°C gelöst und anschließend mit 6 ml 23 gew.-%iger wäßriger Natriumnitritlösung versetzt. Dann wurde 30 min bei 0 bis 5°C nachgerührt bis eine klare Lösung entstanden war.
b) 2,7 g (0,02 mol) 2,3,6-Trimethylphenol in 100 ml Wasser wurden durch Zugabe von 50 gew.-%iger Natronlauge auf einen pH-Wert von 13 gestellt und mit der unter a) beschriebenen Diazoniumsalzlösung versetzt. Nach Rühren über Nacht wurde der pH-Wert mit 10 gew.-%iger Salzsäure auf 2,1 erniedrigt und der ausgefallene Feststoff isoliert.
Ausbeute: 5,8 g

### Beispiel 81 (Herstellung von F7)

a) 7,19 g (0,05 mol) p-Nitranilin und 0,1 g eines Netzmittels wurden in 45 ml Wasser und 17,5 ml 30 gew.-%iger Salzsäure suspendiert. Anschließend wurde mit 55 g Eis und 16,5 ml 23 gew.-%iger wäßriger Natriumnitritlösung versetzt und 1 h bei 0 bis 5°C gerührt.
b) 7,56 g (0,05 mol) 2-Monoethylamino-4-methylphenol wurden in 100 ml Wasser gegeben und mit 50 gew.-%iger Natronlauge auf einen pH-Wert von 10 eingestellt. Dann wurde bei 5°C mit der unter a) beschriebenen Diazoniumsalzlösung versetzt. Der pH-Wert wurde mit 10 gew.-%iger Natronlauge zwischen 7 und 8 gehalten. Nach Rühren über Nacht erniedrigte sich der pH-Wert auf 4,6 und der ausgefallene Feststoff wurde abgesaugt.
Ausbeute: 14,1 g

### Beispiel 82 (Herstellung von F8)

a) 53,3 g (0,05 mol) m-Aminoazotoluol-hydrochlorid (24,5 gew.-%ig) und 0,2 g eines Netzmittels wurden in 40 ml Wasser und 15 ml 30 gew.-%iger Salzsäure suspendiert. Anschließend wurde mit 25 g Eis, 8,4 ml Toluol und 15 ml 23 gew.-%iger wäßriger Natriumnitritlösung versetzt und 2 h bei 5 bis 10°C gerührt.
b) 7,56 g (0,05 mol) 2-Monoethylamino-4-methylphenol wurde in 100 ml Wasser gegeben und mit 50 gew.-%iger Natronlauge ein pH-Wert von 10 eingestellt. Dann wurde bei 5°C mit der unter a) beschriebenen Diazoniumsalzlösung versetzt. Der pH-Wert wurde mit 10 gew.-%iger Natronlauge zwischen 7 und 8 gehalten. Nach Rühren über Nacht wurde der pH-Wert mit Salzsäure auf 1,5 gestellt und der ausgefallene Feststoff abgesaugt.
Ausbeute: 15,9 g

### Beispiel 83 (Herstellung von F9)

a) 42,6 g (0,04 mol) m-Aminoazotoluol-hydrochlorid (24,5 gew.-%ig) und 0,2 g eines Netzmittels wurden in 32 ml Wasser und 12 ml 30 gew.-%iger Salzsäure suspendiert. Anschließend wurde mit 20 g Eis, 7 ml Toluol und 12 ml 23 gew.-%iger wäßriger Natriumnitritlösung versetzt und 2 h bei 5 bis 10°C gerührt.
b) 6,7 g (0,04 mol) m-N,N-Diethylaminophenol wurden in 100 ml Wasser gegeben und mit 50 gew.-%iger Natronlauge ein pH-Wert von 13,2 eingestellt. Dann wurde bei 5°C mit der unter a) beschriebenen Diazoniumsalzlösung versetzt. Der pH-Wert wurde mit 10 gew.-%iger Natronlauge zwischen 4 und 5 gehalten. Nach Rühren über Nacht wurde mit 10 gew.-%iger Salzsäure der pH-Wert auf 1,5 erniedrigt und der ausgefallene Feststoff isoliert.
Ausbeute: 16 g

In analoger Weise werden die Farbstoffe F5 und F6 erhalten.

## Patentansprüche

1. Verfahren zum Nachweis von mit Azofarbstoffen markierten Mineralölen, indem man das markierte Mineralöl mit einem Extraktionsmittel, enthaltend Wasser, ein Lösungsmittel und Base, behandelt, wobei der Azofarbstoff vom Mineralöl in die wäßrige Phase übertritt, dadurch gekennzeichnet, daß man als Markierungsmittel einen Azofarbstoff der Formel Ia 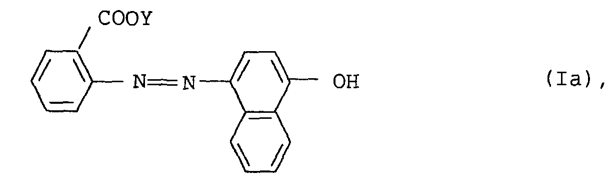 in der
Y C₇-C₁₁-Alkyl bedeutet,
oder der Formel Ib 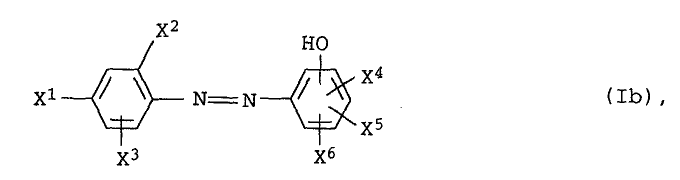 in der
X¹ Wasserstoff oder durch C₁-C₄-Alkyl substituiertes Phenylazo,
X² Wasserstoff,
X³ Wasserstoff oder C₁-C₄-Alkyl,
X⁴ Wasserstoff, C₁-C₄-Alkyl, C₁-C₄-Dialkylamino oder C₁-C₁₆-Monoalkylamino,
X⁵ Wasserstoff oder C₁-C₄-Alkyl und
X⁶ Wasserstoff, C₁-C₄-Alkyl oder C₁-C₄-Alkoxy bedeuten,
mit der Maßgabe, daß a) mindestens einer der drei Reste X¹, X² und X³ von Wasserstoff verschieden ist, b) X² auch Cyano bedeutet, wenn X⁴ für C₁-C₄-Dialkylamino steht, c) X² auch C₁-C₄-Alkoxycarbonyl bedeutet, wenn alle Reste X⁴, X⁵ und X⁶ von Wasserstoff verschieden sind und d) einer der Reste X¹, X² oder X³ auch Nitro bedeutet, wenn X⁴ für C₁-C₁₆-Monoalkylamino und X⁶ für C₁-C₄-Alkyl stehen,
als Lösungsmittel ein mit Wasser teilweise oder vollständig mischbares organisches Lösungsmittel und als Base ein Alkali- oder Erdalkalihydroxid, ein Alkalicarbonat oder eine Ammoniumverbindung der Formel II 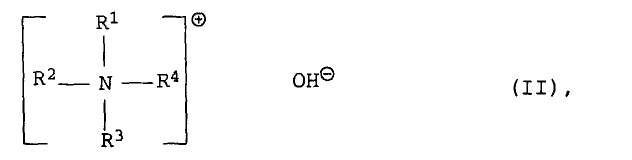 worin R¹, R², R³ und R⁴ unabhängig voneinander jeweils C₁-C₁₆-Alkyl oder Benzyl bedeuten, verwendet.

2. Verfahren nach Anspruch 1, dadurch gekennzeichnet, daß man als Base ein Alkali- oder Erdalkalihydroxid verwendet.

3. Verwendung von Azofarbstoffen der Formel Ia 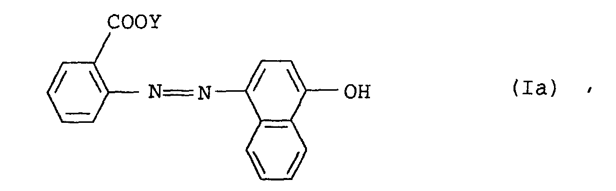 in der
Y C₇-C₁₁-Alkyl bedeutet,
zum Markieren von Mineralölen.

## Claims

1. A method for detecting azo dye marked mineral oils by treating the marked mineral oil with an extractant comprising water, a solvent and a base, the azo dye transferring from the mineral oil into the aqueous phase, which comprises using as marker an azo dye of the formula Ia 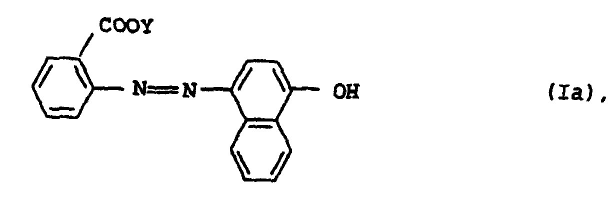 where
Y is C₇-C₁₁-alkyl,
or of the formula Ib 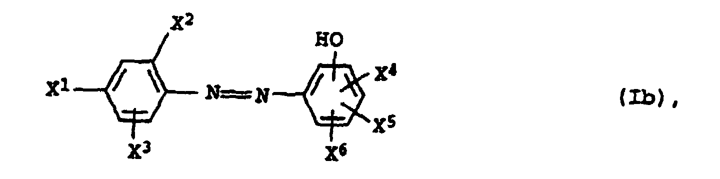 where
X¹ is hydrogen, or C₁-C₄-alkyl-substituted phenylazo,
X² is hydrogen,
X³ is hydrogen or C₁-C₄-alkyl,
X⁴ is hydrogen, C₁-C₄-alkyl, C₁-C₄-dialkylamino or C₁-C₁₆-monoalkylamino,
X⁵ is hydrogen or C₁-C₄-alkyl, and
X⁶ is hydrogen, C₁-C₄-alkyl or C₁-C₄-alkoxy,
with the proviso that a) at least one of the three radicals X¹, X² and X³ is different from hydrogen, b) X² may also be cyano when X⁴ is C₁-C₄-dialkylamino, c) X² may also be C₁-C₄-alkoxycarbonyl when all the radicals X⁴, X⁵ and X⁶ are different from hydrogen, and d) one of the radicals X¹, X² or X³ may also be nitro when X⁴ is C₁-C₁₆-monoalkylamino and X⁶ is C₁-C₄-alkyl,
as solvent a partially or completely water-miscible organic solvent, and as base an alkali or alkaline earth metal hydroxide, an alkali metal carbonate or an ammonium compound of the formula II 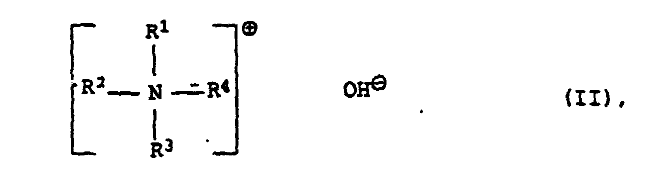 where R¹, R², R³ and R⁴ are each independently of the others C₁-C₁₆-alkyl or benzyl.

2. A method as claimed in claim 1, wherein the base used is an alkali or alkaline earth metal hydroxide.

3. The use of azo dyes of the formula Ia 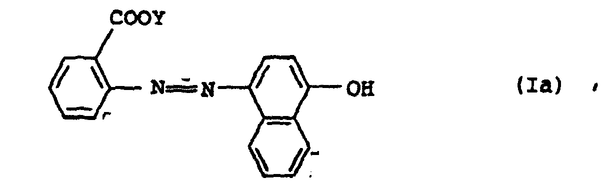 where
Y is C₇-C₁₁-alkyl,
for marking mineral oils.

## Revendications

1. Procédé pour détecter des huiles minérales marquées avec des colorants azoïques, dans lequel on traite l'huile minérale marquée avec un agent d'extraction, contenant de l'eau, un solvant et une base, dans lequel le colorant azoïque de l'huile minérale passe dans la phase aqueuse, caractérisé en ce que l'on utilise comme agent de marquage un colorant azoïque de formule Ia 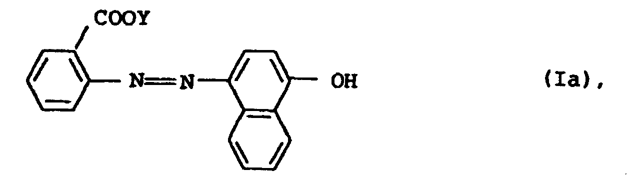 dans laquelle
Y représente un groupe alkyle en C₇ à C₁₁,
ou de formule Ib 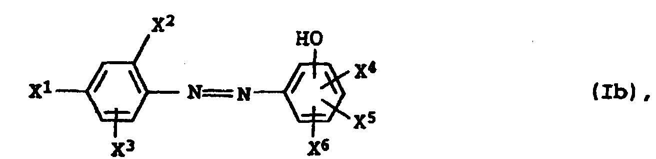 dans laquelle
X¹ représente un atome d'hydrogène ou un groupe phénylazoïque à substitution alkyle en C₁ à C₄,
X² représente un atome d'hydrogène,
X³ représente un atome d'hydrogène ou un groupe alkyle en C₁ à C₄,
X⁴ représente un atome d'hydrogène, un groupe alkyle en C₁ à C₄, dialkylamino en C₁ à C₄ ou monoalkylamino en en C₁ à C₁₆,
X⁵ représente un atome d'hydrogène ou un groupe alkyle en C₁ à C₄, et
X⁶ représente un atome d'hydrogène, un groupe alkyle en C₁ à C₄ ou alcoxy en C₁ à C₄, avec la condition que a) au moins l'un des trois résidus X¹, X², X³ est différent de l'atome d'hydrogène, b) X² représente également un groupe cyano, lorsque X⁴ est mis pour un groupe dialkylamino en C₁ à C₄, c) X² représente également un groupe alcoxycarbonyle en C₁ à C₄, si tous les résidus X⁴, X⁵ et X⁶ sont différents de l'atome d'hydrogène et d) un des résidus X¹, X² ou X³ représentent également un groupe nitro, lorsque X⁴ est mis pour un groupe alkylamino en C₁ à C₁₆ et X⁶ pour un groupe alkyle en C₁ à C₄,
comme solvant, un solvant organique partiellement ou complètement miscible à l'eau et comme base, un hydroxyde de métal alcalin ou de métal alcalino-terreux, un carbonate de métal alcalin ou un composé d'ammonium de formule II 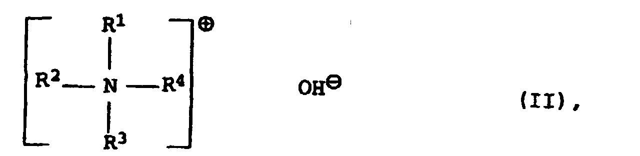 dans laquelle R¹, R², R³ et R⁴ représentent chacun indépendamment l'un de l'autre un groupe alkyle en C₁ à C₁₆ ou benzyle.

2. Procédé selon la revendication 1, caractérisé en ce que l'on utilise comme base, un hydroxyde de métal alcalin ou de métal alcalino-terreux.

3. Utilisation de colorants azoïques de formule Ia 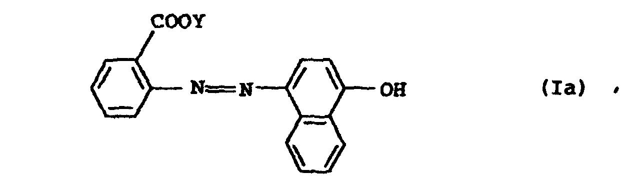 dans laquelle Y représente un groupe alkyle en C₇ à C₁₁ pour marquer des huiles minérales.
